Europäisches Patentamt

(19) European Patent Office

Office européen des brevets

(11) Numéro de publication : **0 224 487**
**B1**

(12) **FASCICULE DE BREVET EUROPÉEN**

(45) Date de publication du fascicule du brevet :
**17.05.89**

(21) Numéro de dépôt : **85904260.8**

(22) Date de dépôt : **29.08.85**

(86) Numéro de dépôt international :
**PCT/FR 85/00233**

(87) Numéro de publication internationale :
**WO/8601390 (13.03.86 Gazette 86/06)**

(51) Int. Cl.⁴ : **A 61 B   1/12**

(54) **APPAREIL D'IRRIGATION CONTROLEE DES CAVITES ET CONDUITS NATURELS DU CORPS HUMAIN.**

(30) Priorité : 31.08.84 FR 8413550
31.08.84 FR 8413551

(43) Date de publication de la demande :
**10.06.87 Bulletin 87/24**

(45) Mention de la délivrance du brevet :
**17.05.89 Bulletin 89/20**

(84) Etats contractants désignés :
**AT BE CH DE FR GB IT LI LU NL SE**

(56) Documents cités :
**EP–A– 0 112 585**
**WO–A–82 /005 90**
**DE–A– 2 611 698**
**FR–A– 2 378 394**
**FR–A– 2 508 671**
**US–A– 3 900 022**

(73) Titulaire : BURNER, Robert
**21 rue Damberg**
**F-68200 Brunstatt (FR)**

(72) Inventeur : BURNER, Robert
**21 rue Damberg**
**F-68200 Brunstatt (FR)**

(74) Mandataire : NITHARDT, Roland
**CABINET ROLAND NITHARDT 12, rue du 17 Novembre B.P. 1445**
**F-68071 Mulhouse Cedex (FR)**

Jouve, 18, rue St-Denis, 75001 Paris, France

**Description**

La présente invention concerne un appareil d'irrigation contrôlée des cavités et conduits naturels du corps humain, notamment pour endoscope, en particulier pour endoscope urologique, comportant au moins un canal pour injecter soit de façon intermittente, soit de façon continue, un liquide d'irrigation dans une cavité et/ou un conduit corporel dits zone d'intervention, de manière à permettre aussi bien des interventions en irrigation intermittente qu'en irrigation continue, cet appareil comportant un réservoir contenant le liquide d'irrigation, un conduit de liaison pour raccorder ce réservoir audit canal, une pompe d'alimentation montée sur le conduit de liaison pour approvisionner ledit canal en liquide d'irrigation, des premiers moyens sensibles à la pression raccordés audit conduit de liaison en aval de la pompe pour mesurer la pression instantanée régnant dans ce conduit au niveau de ces moyens sensibles à la pression et pour émettre un signal de mesure représentatif de cette pression, ainsi que des moyens pour définir une pression maximale acceptable dans ladite zone d'intervention.

On sait qu'en endoscopie certaines interventions, qu'elles soient purement exploratoires ou qu'elles soient opératoires, nécessitent la présence de liquide, en général à base d'eau, dans la zone d'intervention. Il en est ainsi, en particulier en endoscopie urologique, notamment pour la vessie, les uretères, l'urètre, la prostate ou les reins. Mais cette nécessité se rencontre également, plus généralement, dans le cas de cavités naturelles ou pathologiques, à explorer ou à traiter par exemple par résection ou par simple lavage ou irrigation.

En endoscopie en milieu liquide artificiel, on pratique soit sous irrigation continue, soit sous irrigation intermittente en présence ou en l'absence du flux liquidien.

Dans le premier cas, le liquide est injecté par un canal de l'endoscope et extrait soit par un autre canal de cet endoscope, soit par un moyen séparé du genre cathéter. Dans le second cas, c'est le même canal de l'endoscope qui, cycliquement, sert à l'injection et à l'extraction du liquide.

Que ce soit en irrigation continue ou en irrigation intermittente, l'injection de liquide se fait actuellement par gravité à l'aide d'un conteneur de liquide surélevé par rapport à l'endoscope en position de travail. Il s'ensuit que la pression du liquide injecté est, dans la zone d'intervention, déterminée en principe par la hauteur de la colonne de liquide entre l'endoscope et le niveau du liquide dans le conteneur.

En irrigation intermittente, l'évacuation du liquide se fait actuellement sous la commande manuelle de l'opérateur qui, soit ouvre un robinet d'évacuation ou similaire monté sur la gaine de l'endoscope, soit extrait l'ensemble bloc optique-bloc opérateur de la gaine de l'endoscope. Cette méthode ne présente pas d'inconvénients majeurs puisqu'il s'agit simplement de vider le champ exploratoire ou zone d'intervention.

En irrigation continue, l'évacuation du liquide se fait actuellement à travers le canal d'évacuation en cas d'utilisation d'un endoscope à double courant, soit à travers un cathéter en cas d'utilisation d'un endoscope monocourant. Dans les deux cas, elle est réalisée soit au moyen de la gravité du liquide à évacuer, soit par une pompe à débit sélectionné mais constant.

Une première difficulté provient du fait que le niveau du liquide à injecter contenu dans le conteneur est variable dans le temps en raison de la consommation de liquide. Il faut donc soit s'accommoder de la variation de pression qui en résulte, soit relever d'autant le conteneur.

Une seconde difficulté résulte, en irrigation continue, du fait qu'il n'est pas toujours aisé de réguler parfaitement l'extraction du liquide. Une extraction à débit trop important fait baisser la pression dans la zone de l'intervention, tandis qu'une extraction à débit trop faible risque de créer une surpression dangereuse dans cette zone.

La pression qui règne dans la zone d'intervention est en relation directe avec le remplissage de cette zone, c'est-à-dire avec le volume qui y est contenu. Un remplissage incomplet peut nuire à la qualité de l'exploration et rendre dangereuse la phase opératoire, tandis qu'un remplissage excessif engendrant une pression excessive peut être dangereux pour le patient, surtout en cas d'opération. Par ailleurs, si le débit d'évacuation est ou devient supérieur au débit d'injection, le remplissage du champ opératoire est incomplet, ce qui engendre les premiers inconvénients mentionnés ci-dessus. Si au contraire, le débit d'évacuation est ou devient inférieur au débit d'injection, le remplissage du champ opératoire est excessif, ce qui provoque une pression excessive dans la zone d'intervention et engendre les seconds inconvénients mentionnés précédemment.

Le praticien doit donc veiller attentivement à ce que la pression du liquide ne soit ni trop importante ni trop faible en contrôlant simultanément l'injection et l'évacuation du liquide d'irrigation. Ces contrôles constituent pour lui une tâche venant s'ajouter aux opérations toujours délicates de l'intervention endoscopique proprement dite. En plus, les systèmes d'irrigation conventionnels ne fournissent au praticien qu'une connaissance très approximative, voire aléatoire, du débit instantané du volume de liquide injecté et du volume de liquide évacué, ce qui empêche pratiquement tout contrôle précis de ces divers paramètres.

La présente invention a pour but de remédier à ces divers inconvénients en proposant un appareil d'irrigation contrôlée tel que mentionné ci-dessus qui, en irrigation continue, assure un travail à pression constante aussi basse que possible et qui, en irrigation intermittente, assure un travail à pression

EP 0 224 487 B1

croissante mais limitée au-dessous d'un seuil qui ne peut être dépassé.

Ce but est atteint par l'appareil selon l'invention caractérisé en ce qu'il comporte des moyens de correction pour pondérer ledit signal de mesure représentatif de la pression en fonction d'une part, des pertes de charge et d'autre part de la différence de niveau qui existent entre lesdits moyens sensibles à la pression et ladite zone d'intervention pour déterminer la valeur de la pression instantanée au niveau de la zone d'intervention, et des moyens pour contrôler l'injection de liquide d'irrigation de telle manière que ladite pression dans la zone d'intervention soit à chaque instant inférieure ou égale à ladite pression maximale.

Selon une forme de réalisation préférée, lesdits moyens pour pondérer le signal en fonction desdites pertes de charge comportent une unité de traitement des signaux agencée pour recevoir un signal de consigne de débit d'injection d'un circuit sélecteur du débit d'injection et pourvu d'un organe de réglage, cette unité de traitement de signaux étant également agencée pour recevoir un signal de débit transmis par un élément activé par le liquide propulsé par la pompe et pour fournir un signal de correction au circuit correcteur, ce signal de correction étant fonction du débit d'injection mesuré régulé selon ledit signal de consigne de débit.

Selon cette même forme de réalisation préférée de l'appareil selon l'invention, lesdits moyens pour pondérer le signal en fonction desdites pertes de charge comportent un circuit de réglage et de sélection du débit d'injection, pourvu d'un organe de réglage, ce circuit et cet organe de réglage étant agencés pour délivrer des signaux distincts à une unité de commande de la pompe d'alimentation, ces signaux distincts correspondant à des valeurs différentes du débit de liquide injecté dans la zone d'intervention, ces valeurs différentes étant affichées au moyen dudit organe de réglage.

Toujours selon ce mode de réalisation préférée, lesdits moyens pour pondérer le signal en fonction de la différence de niveau comportent un circuit correcteur couplé à un circuit réglable de correction pourvu d'un organe de réglage, ce circuit réglable de correction et cet organe de réglage étant agencés pour délivrer au circuit correcteur des signaux distincts correspondant respectivement à des différences de niveau distinctes entre lesdits moyens sensibles à la pression et ladite zone d'intervention, ces différences de niveau distinctes étant affichées au moyen dudit organe de réglage.

Selon la forme de réalisation préférée de l'appareil de l'invention, lesdits moyens pour définir une pression maximale acceptable dans la zone d'intervention comportent un circuit de réglage pourvu d'un organe de réglage, ce circuit et cet organe de réglage étant agencés pour délivrer à une première entrée d'un comparateur des signaux distincts correspondant respectivement à différentes valeurs de ladite pression maximale acceptable dans la zone d'intervention, ces différentes valeurs étant affichées au moyen dudit organe de réglage.

Selon un mode de réalisation particulièrement avantageux, l'appareil comporte différents dispositifs d'affichage lumineux respectivement conçus pour visualiser la pression instantanée dans la zone d'intervention, la pression instantanée mesurée par lesdits premiers moyens sensibles à la pression et la pression instantanée dans le canal intérieur de l'endoscope.

L'appareil selon l'invention comporte par ailleurs de préférence un élément agencé pour émettre un signal représentatif du débit de liquide injecté et une unité de traitement connectée à au moins un compteur de volume du liquide délivré par la pompe et au circuit correcteur.

Selon une forme préférentielle, l'appareil comporte deux compteurs dont le premier est agencé pour mesurer le volume de liquide délivré depuis le début d'un cycle en cours, en cas d'une irrigation intermittente, et dont le second est agencé pour mesurer le volume de liquide délivré depuis le début de l'intervention quel que soit le mode d'irrigation.

Selon un mode de réalisation particulièrement avantageux, l'appareil comporte un circuit de sélection pourvu d'un organe de réglage agencé pour émettre des signaux à un comparateur, ces signaux correspondant à différents volumes de liquide à délivrer pendant une intervention ou un cycle en cours, ces différents volumes étant sélectionnés au moyen dudit organe de réglage.

Dans ce cas, le comparateur peut comporter une première entrée pour lesdits signaux provenant du circuit de sélection et correspondant auxdits différents volumes de liquide, et une seconde entrée pour des signaux émis par ledit premier compteur.

Chacun desdits premier et second compteur est de préférence respectivement associé à un premier et un second dispositif d'affichage lumineux.

Pour permettre d'arrêter l'injection de liquide dans la zone d'intervention lorsqu'un volume prédéterminé de ce liquide a été injecté, l'appareil, selon un mode de réalisation préféré, comporte un premier et un second dispositif de remise à zéro, ces dispositifs étant respectivement associés au premier compteur et au second compteur, le premier dispositif de remise à zéro étant agencé pour agir sur un comparateur couplé à une unité de commande d'une électrovanne interposée sur le conduit.

Afin de raccorder le dispositif d'injection de l'appareil d'irrigation selon l'invention à un dispositif d'évacuation actif contrôlé, il comporte deux circuits d'interface conçus pour amplifier et adapter respectivement les signaux représentatifs du débit et de la pression instantanée dans la zone d'intervention, ainsi que deux bornes de raccordement pour connecter lesdits circuits d'interface.

Selon une forme de mise en œuvre particulièrement intéressante, l'appareil d'irrigation selon l'invention, dans lequel ledit dispositif d'évacuation actif contrôlé comporte un conduit pour évacuer le liquide de la zone d'intervention, une pompe d'évacuation montée sur ce conduit, et des seconds moyens sensibles à la pression raccordés audit conduit en aval de ladite zone d'intervention, est caractérisé en ce que ledit dispositif comporte des moyens de correction pour pondérer le signal de mesure émis par lesdits seconds moyens sensibles à la pression en fonction d'une part des pertes de charge et d'autre part de la différence de niveau qui existent entre lesdits seconds moyens sensibles à la pression et ladite zone d'intervention et des moyens pour contrôler l'évacuation de liquide d'irrigation de telle manière que ladite pression dans la zone d'intervention soit à chaque instant inférieure ou égale à ladite pression maximale.

Dans ce cas, lesdits moyens de correction pour pondérer le signal de mesure émis par lesdits· seconds moyens sensibles à la pression en fonction des pertes de charge comportent de préférence une unité de comparaison et de traitement et un circuit de correction équipé d'un organe de réglage, ce circuit et cet organe étant agencés pour délivrer à l'unité des signaux distincts correspondant respectivement à des sections hydrauliques équivalentes différentes des conduits d'évacuation, ces sections hydrauliques équivalentes différentes étant sélectionnées au moyen dudit organe de réglage.

Dans ce cas également, lesdits moyens pour pondérer le signal en fonction desdites pertes de charge comportent de préférence un circuit correcteur pourvu d'un organe sélecteur, ce circuit correcteur et cet organe sélecteur étant agencés pour sélectionner les débits prédéterminés de liquide évacué de la zone d'intervention. Ils comportent également une borne de liaison connectée à un circuit de correction équipé d'un organe de réglage agencé pour prendre en compte un signal représentatif du débit instantané du dispositif d'injection.

Dans ce cas enfin, lesdits moyens pour pondérer le signal en fonction de la différence de niveau comportent de préférence un circuit de réglage de correction pourvu d'un organe sélecteur, ce circuit et cet organe étant agencés pour délivrer à un circuit correcteur des signaux distincts correspondant respectivement à des différences de niveau distinctes entre lesdits seconds moyens sensibles à la pression et la zone d'intervention, ces différences de niveau distinctes étant sélectionnées au moyen dudit organe sélecteur.

La présente invention sera mieux comprise en référence à la description d'exemples de réalisation et du dessin annexé dans lequel :

La figure 1 représente une vue schématique d'une forme de réalisation préférée du dispositif d'injection de l'appareil d'irrigation selon l'invention, et

La figure 2 représente une vue schématique d'une forme de réalisation préférée du dispositif d'évacuation du dispositif d'irrigation selon l'invention.

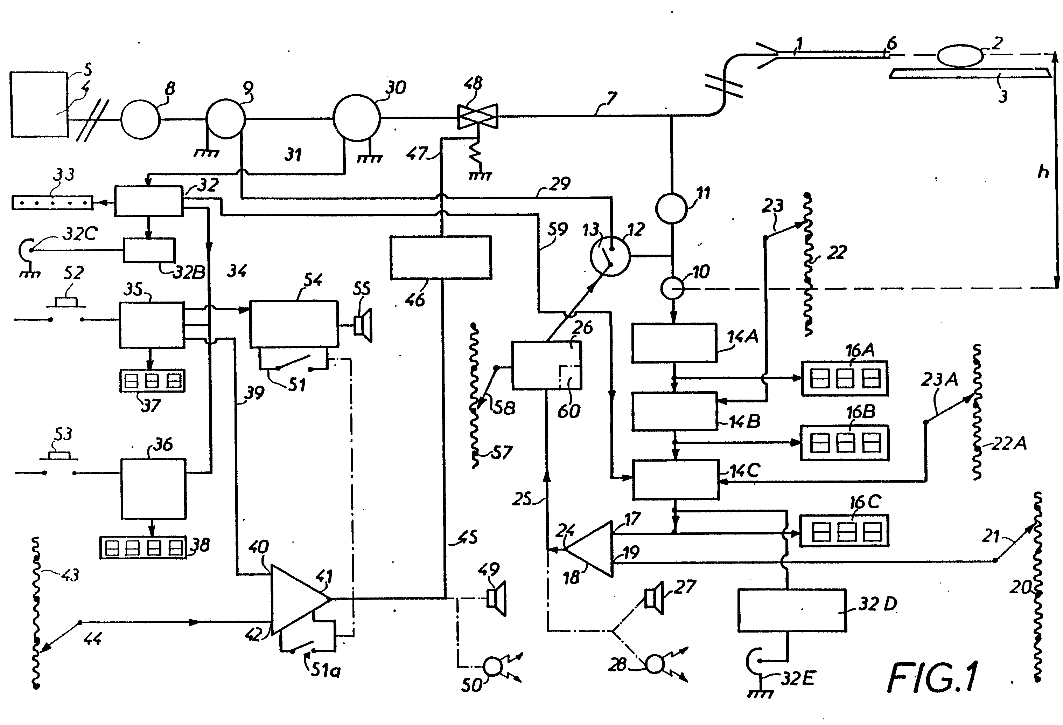

En référence à la fig. 1, le dispositif représenté comporte un endoscope 1 destiné à l'examen et/ou à l'opération d'un organe 2 en forme de poche tel que par exemple la vessie d'un patient reposant sur un support 3. Comme mentionné précédemment, pour ce genre d'intervention il est nécessaire de remplir la poche de la vessie d'un liquide d'irrigation 4, initialement contenu dans un conteneur 5, qui est introduit dans la vessie par un canal intérieur 6 de l'endoscope à travers un conduit 7 équipé d'un filtre 8.

Le liquide d'irrigation 4 peut être délivré à l'endoscope 1 et par conséquent injecté dans l'organe 2, au cours de l'intervention, soit de façon continue, soit de façon intermittente.

Cette injection s'effectue au moyen d'une pompe 9, par exemple du type à engrenages à entraînement magnétique, montée sur le conduit 7 entre l'endoscope et le filtre et qui est destinée à délivrer de façon contrôlée le liquide à l'endoscope. Entre l'endoscope et la pompe, le conduit 7 porte un manomètre 10, de préférence hydrauliquement isolé du liquide 4 par un séparateur 11, par exemple du type à membrane ou similaire, de même qu'un manomètre de sécurité 12 à tarage variable commandant l'ouverture d'un interrupteur 13 au-delà d'une pression maximale de sécurité.

Le manomètre 10 a pour but de mesurer la pression instantanée régnant à son niveau dans le conduit 7 et d'émettre un signal de mesure approprié, tandis que le manomètre 12 est agencé pour laisser l'interrupteur 13 fermé tant que cette pression instantanée est inférieure à ladite pression maximale de sécurité dont la valeur est préréglée.

Le dispositif comporte par ailleurs une unité de traitement 14A conçue pour mettre en forme le signal de mesure émis par le manomètre 10. Son signal de sortie est dirigé, d'une part sur un dispositif d'affichage lumineux 16A destiné à permettre au praticien de visualiser la pression instantanée régnant au niveau du manomètre 10 et d'autre part sur un circuit correcteur 14B conçu pour engendrer un signal de correction au moyen d'un circuit réglable de correction 22 pourvu d'un curseur 23. Grâce à ce circuit réglable de correction, le praticien peut régler l'amplitude du signal de correction délivré au circuit correcteur 14B, cette amplitude étant représentative de la différence de niveau h entre le manomètre 10 et la zone d'intervention définie par l'organe 2, ou plus précisément par l'extrémité de l'endoscope 1. A titre d'exemple, on peut admettre que le circuit réglable de correction soit conçu pour fournir sept valeurs discrètes de correction correspondant à des différences de niveau échelonnées entre 0 et 30 cm.

Le signal de sortie du circuit correcteur 14B est dirigé, d'une part sur un dispositif d'affichage lumineux 16B conçu pour permettre au praticien de visualiser la pression instantanée dans le conduit 7 au niveau de l'endoscope et d'autre part sur un circuit correcteur 14C qui reçoit un premier signal de correction provenant d'un circuit réglable de correction 22A pourvu par exemple d'un curseur 23A prévu pour permettre au praticien de régler l'amplitude du signal de correction délivré au circuit correcteur 14C, cette amplitude étant représentative de la section du canal intérieur 6 de l'endoscope. A titre d'exemple, le circuit réglable de correction 22A est conçu pour permettre de délivrer trois valeurs discrètes de correction correspondant à des sections hydrauliques équivalentes à 3, 5 et 7 mm². Le circuit correcteur

4

14C est par ailleurs conçu pour recevoir, au travers d'une ligne 59, un second signal de correction provenant d'une unité 32, qui sera décrite par la suite, et qui délivre sur ladite ligne 59 un signal représentatif du débit d'injection.

Il en résulte que dans le circuit 14C, le signal de mesure de la pression, déjà corrigé en fonction de la différence de niveau entre le point de mesure et l'endoscope, est ainsi corrigé en fonction de la section de passage du liquide 4 dans le canal intérieur 6 de l'endoscope et en fonction du débit d'injection. En d'autres termes, le signal de mesure de la pression est corrigé en fonction de la perte de charge résultant de ces deux facteurs.

Le signal de sortie du circuit correcteur 14C est dirigé d'une part sur un dispositif d'affichage lumineux 16C conçu pour permettre au praticien de visualiser la pression ainsi pondérée et d'autre part sur une entrée 17 d'un circuit comparateur 18 dont l'autre entrée 19 reçoit un signal de référence provenant d'un circuit de réglage schématisé en 20, grâce auquel le praticien peut régler, à l'aide d'un curseur ou similaire 21, la pression maximale admissible dans la zone d'intervention définie par l'organe 2, pendant l'intervention, et modifier de ce fait les caractéristiques du signal délivré à l'entrée 19. A titre d'exemple, on peut prévoir un réglage de la pression maximale admissible dans la zone d'intervention selon douze valeurs discrètes allant de 5 à 60 cm de colonne d'eau. A titre de variante, on pourrait également prévoir un fonctionnement du circuit comparateur 18 tel que le débit soit régulé de telle manière que la pression dans la zone d'intervention ne dépasse pas une valeur prédéterminée sélectionnée par l'opérateur. A cet effet, le praticien sélectionne ladite pression grâce au circuit de réglage 20, à l'aide du curseur 21.

Le dispositif d'affichage 16C est particulièrement utile au praticien puisqu'il permet à celui-ci de surveiller la pression réelle régnant dans l'organe 2. En outre, les afficheurs 16A et 16B constituent pour lui des moyens de contrôle très efficaces puisqu'ils lui permettent de surveiller en permanence la pondération liée à la différence de niveau h définie précédemment.

Grâce aux corrections liées, d'une part à la différence de niveau h entre le manomètre et l'endoscope, d'autre part à la section d'entrée de l'endoscope et enfin au débit d'injection, l'opérateur peut selon les besoins modifier la hauteur de travail, changer l'endoscope et/ou faire varier le débit sans avoir à se préoccuper d'une éventuelle variation de pression incontrôlée dans l'organe 2. Ces corrections permettent également de connaître, grâce à une mesure effectuée à l'extérieur de l'organe 2, et ceci avec une précision suffisante, la pression régnant effectivement à l'intérieur de l'organe 2 sans avoir à se livrer à un quelconque calcul et sans être contraint de modifier l'endoscope en l'équipant d'un système de détection de pression.

Le signal de comparaison apparaissant à la sortie 24 du comparateur 18, est acheminé par une ligne 25 vers une unité 26 de commande de la pompe et, éventuellement, vers des moyens de signalisation sonore 27 et/ou lumineuse 28. Ces moyens de signalisation sont prévus pour émettre un signal lorsque la pression maximale sélectionnée est atteinte. L'unité de commande 26 actionne un circuit 29 d'alimentation de la pompe 9 dans lequel est interposé l'interrupteur 13. Le cas échéant, on peut prévoir des moyens correcteurs additionnels 60 liés à la température des moyens sensibles à la pression, cette température étant une fonction de la température ambiante, notamment de celle du liquide et de celle du manomètre 12.

L'unité de commande·26 est associée à un circuit 57 de réglage et de sélection du débit d'injection. Ce circuit réglable peut comporter un curseur 58 grâce auquel le praticien peut régler l'amplitude du signal de commande, cette amplitude étant représentative du débit le mieux adapté à une intervention donnée. On peut prévoir différentes valeurs de réglage du débit, ces valeurs étant par exemple comprises entre 0 et 700 cm³/mn.

En fonctionnement, tant que la pression régnant dans le conduit 7 et, moyennant les corrections ci-dessus, dans l'organe 2, est inférieure à la pression maximale de référence affichée dans le circuit de sélection 20, la pompe est excitée et le liquide est délivré à l'endoscope pour être injecté dans ledit organe. Quand la pression maximale de référence est atteinte, le comparateur 18 commande l'unité 26 qui met la pompe à l'arrêt, ce qui provoque l'arrêt de l'alimentation de l'endoscope en liquide. Si pour une raison quelconque la pression dans le conduit 7 atteignait la valeur maximale de sécurité sur laquelle est taré le manomètre 12, l'interrupteur 13 provoquerait l'arrêt de la pompe 9.

Le dispositif décrit comporte, en outre, avantageusement des moyens additionnels agencés pour mesurer le volume du liquide injecté depuis le début d'une intervention en cours. Ces moyens additionnels sont particulièrement utiles dans le cas d'interventions sous irrigation intermittente. Dans l'exemple décrit, ces moyens sont pilotés par un élément 30 monté en série sur le conduit 7 en aval de la pompe 9. L'élément 30 est actionné par le liquide propulsé par la pompe et émet un signal représentatif du débit, par exemple des impulsions dont la fréquence est proportionnelle au débit. A titre de variante, l'élément 30 pourrait se présenter sous la forme d'engrenages et pourrait être intégré à la pompe 9. Les impulsions fournies par l'élément 30, dont chacune représente un volume unitaire, sont transmises par une ligne 31 à une unité 32 de traitement de signaux qui commande un dispositif d'affichage lumineux 33, du type à diodes électroluminescentes ou similaires, qui permet au praticien de visualiser le débit instantané. L'unité 32 commande également, à travers sa ligne de sortie 34, un premier et un second compteur 35 et 36. Chacun des compteurs 35 et 36 est respectivement associé à un afficheur lumineux 37 et 38 conçu pour permettre au praticien de visualiser les volumes calculés par comptage des impulsions

EP 0 224 487 B1

émises par l'élément 30 et mises en forme par l'unité 32.

Le premier compteur 35 est destiné à mesurer le volume de liquide délivré depuis le début du cycle en cours, dans le cas d'une irrigation intermittente. Ce même compteur 35 pilote un élément 54 agencé pour délivrer, par l'intermédiaire d'un dispositif sonore 55, des sons par exemple conformément au mode suivant :

a) si un inverseur manuel 51 qui commande l'élément 54 est sur la position « volume limité » :

de   0 à 200 cm³ : un signal sonore/10 cm³ )
de 200 à 300 cm³ : deux signaux sonores/10 cm³ } Tonalité basse
de 300 à 400 cm³ : trois signaux sonores/10 cm³ )

de 400 à 500 cm³ : un signal sonore/10 cm³ }
de 500 à 600 cm³ : deux signaux sonores/10 cm³ } Tonalité haute

b) si l'inverseur 51 est sur la position « volume illimité » : un signal sonore/10 cm³.

Le compteur 36 est destiné à mesurer le volume de liquide délivré depuis le début de l'intervention quel que soit le mode d'irrigation, c'est-à-dire continu ou intermittent.

Le compte du compteur 35 est acheminé par une ligne 39 vers une entrée 40 d'un circuit comparateur 41 dont l'autre entrée 42 reçoit un signal de référence provenant d'un circuit de réglage, schématisé en 43, grâce auquel le praticien règle à l'aide d'un curseur ou similaire 44, le volume de liquide à délivrer pendant l'intervention ou le cycle en cours. A titre d'exemple, on peut prévoir dix valeurs discrètes de volumes à injecter s'échelonnant entre 150 et 600 cm³.

Le signal issu de la comparaison est acheminé par une ligne 45 vers une unité 46 commandant la ligne d'alimentation 47 d'une électrovalve 48 montée sur le conduit 7. Tant que le volume mesuré par le compteur 35 est inférieur au volume préréglé dans le circuit de sélection 43, le signal de comparaison laisse l'électrovalve 48 ouverte. Au contraire, dès que le volume mesuré égale ce volume préréglé, il commande la fermeture de l'électrovalve 48, ce qui a pour effet d'interrompre l'alimentation de l'endoscope en liquide. Le signal de comparaison transmis par la ligne 45 peut commander des moyens de signalisation sonore 49 ou lumineuse 50 qui émettent un signal dès que le volume maximal présélectionné est atteint.

Selon une forme de réalisation préférée, l'unité de commande 26 et l'unité de commande 46 peuvent être confondues de telle manière que la pompe et l'électrovalve soient commandées simultanément.

Il va de soi que cette comparaison entre le volume délivré et le volume de référence n'a de signification que pour les interventions sous irrigation intermittente. C'est pourquoi, pour les interventions sous irrigation continue, on prévoit un inverseur 51a qui est couplé à un inverseur manuel 51 et qui, dans l'une de ses deux positions correspondant à l'irrigation continue, active en permanence l'unité 46 par le comparateur 41 pour commander l'électrovalve 48 afin de laisser celle-ci ouverte et qui, dans l'autre position correspondant à l'irrigation intermittente, fait commander l'unité 46 par le comparateur 41.

Chacun des compteurs 35 et 36 est respectivement équipé d'un dispositif associé 52 et 53 de remise à zéro qui peut être actionné automatiquement à la mise en marche du dispositif ou manuellement par le praticien. L'injection de liquide a lieu tant que le volume délivré postérieurement à la remise à zéro précédente est inférieur au volume maximal présélectionné, par exemple 400 cm³. Au moment où ce volume atteint cette valeur, soit par exemple 400 cm³, l'injection est arrêtée automatiquement et ne pourra reprendre qu'après une nouvelle remise à zéro par le bouton 52, après l'évacuation du liquide de la zone d'intervention. En outre, le praticien est en mesure d'arrêter l'injection à tout instant avant que le volume n'atteigne cette valeur.

Il ressort de la description précédente que l'appareil décrit ci-dessus permet d'effectuer une présélection de la pression maximale d'intervention, fournit un contrôle visuel et/ou sonore de la pression instantanée dans la zone d'intervention et autorise un réglage de cette pression maximale au cours de l'intervention ainsi qu'une action automatique sur la pompe d'alimentation par le praticien lui-même sans l'assistance d'un tiers.

En outre, dans sa réalisation préférée comportant les moyens de mesure du volume de liquide délivré, le dispositif permet une présélection du volume à délivrer par cycle dans le cas d'une irrigation intermittente, un contrôle visuel et/ou sonore à tout moment du volume et du débit délivrés, un réglage de ce volume de référence pendant l'intervention ainsi qu'une action automatique d'interruption du flux de liquide, quand le volume de référence est atteint.

A titre de variante, il serait envisageable de faire tourner la pompe 9 en permanence et de prévoir une vanne 48 du type trois-voies équipée d'un conduit de retour au conteneur 5, cette vanne étant également commandée par l'unité 26 de commande de la pompe.

Pour l'évacuation du liquide à partir de l'organe 2, en irrigation continue ou intermittente, on peut faire appel à divers moyens appropriés, inertes ou actifs, régulés ou non. Toutefois, une sécurité supplémentaire appréciable est obtenue par l'utilisation de moyens d'évacuation actifs contrôlés qui seront décrits plus en détail ci-dessous. A cet effet, le dispositif ci-dessus est équipé de circuits

6

EP 0 224 487 B1

d'interface 32B et 32D conçus pour amplifier et adapter respectivement un des signaux issus de l'unité 32 ayant fonction de débimètre et l'information de pression instantanée de la zone d'intervention fournie par le circuit 14C, ces moyens étant conçus pour alimenter des bornes 32C et 32E de raccordement pour la prise en compte de ces informations.

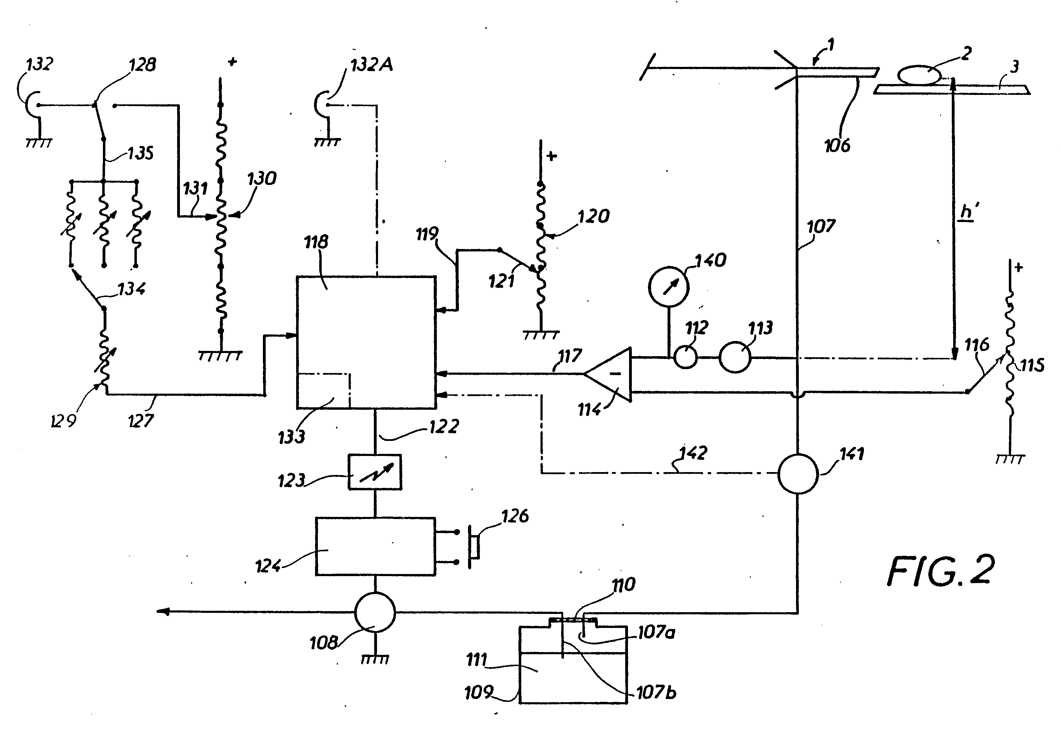

Le dispositif d'évacuation de l'appareil d'irrigation dont le dispositif d'injection est décrit ci-dessus sera présenté plus en détail en référence à la figure 2.

Dans l'exemple représenté, le dispositif d'évacuation comporte un conduit d'évacuation 107 raccordé à un second canal intérieur 106 de l'endoscope 1. Toutefois, dans certains cas, le conduit d'évacuation 107 pourrait être connecté à un cathéter (non représenté) qui débouche à l'intérieur de l'organe 2.

Ce dispositif comporte d'autre part une pompe 108, par exemple péristaltique, qui est montée sur le conduit 107. Dans la pratique, un bocal intermédiaire 109 est interposé sur le conduit 107 en amont de la pompe 108 de telle manière que les branches 107a et 107b respectivement d'arrivée et de départ du conduit 107 traversent de façon hermétique un bouchon 110 de ce bocal. L'extrémité de la branche d'arrivée 107a est de préférence située au-dessus du niveau de liquide 111 tandis que l'extrémité de la branche de départ 107b est située dans la zone supérieure de ce liquide qui constitue ainsi une réserve.

Un manomètre 112, hydrauliquement isolé du liquide d'irrigation par un séparateur 113, par exemple du type à membrane, est monté sur le conduit 107, en dérivation entre l'endoscope 1 et le bocal intermédiaire 109. Ce manomètre 112 est agencé pour mesurer la pression instantanée qui règne à son niveau dans le conduit d'évacuation 107.

Selon une propriété particulièrement avantageuse du dispositif, le signal engendré par le manomètre 112, et qui est représentatif de la pression régnant dans le conduit 107 au niveau de ce manomètre, est corrigé de manière à être fidèlement représentatif de la pression régnant à l'intérieur de l'organe 2. Dans ce but, le dispositif comporte un circuit correcteur 114, par exemple un soustracteur, conçu pour soustraire du signal de mesure délivré par le manomètre 112 un signal de correction provenant d'un circuit réglable de correction 115, par exemple du type potentiométrique, qui permet au praticien de régler, à l'aide d'un curseur 116 ou similaire, l'amplitude du signal de correction délivré au circuit correcteur 114, cette amplitude étant représentative de la différence h' de niveau existant entre l'endoscope 1 et le manomètre 112. A titre d'exemple, l'échelle de correction peut comporter sept valeurs discrètes correspondant à des différences de niveau s'échelonnant de 100 à 130 cm.

Comme dans le cas du dispositif d'injection décrit en référence à la fig. 1, le praticien peut, selon les besoins, modifier la hauteur de travail sans avoir à se préoccuper d'une éventuelle variation de pression engendrée à l'intérieur de l'organe 2 et consécutive à cette modification de la hauteur de travail. Cette propriété du dispositif permet également, par une mesure de pression extérieure à l'organe 2 et par une correction appropriée, de connaître avec une précision suffisante la pression régnant effectivement à l'intérieur de l'organe 2 sans avoir à se livrer à des calculs et sans être contraint de modifier l'endoscope en l'équipant par exemple d'un système de détection de pression. En d'autres termes, le praticien peut utiliser des endoscopes courants tout en bénéficiant de perfectionnements réels et d'une grande précision de travail.

Le signal de sortie du circuit correcteur 114 est transmis par une ligne 117 et acheminé vers une unité 118 de comparaison, de correction et de traitement. Cette unité reçoit également sur une ligne d'entrée 119 un signal de référence provenant d'un circuit de réglage 120, grâce auquel le praticien est en mesure de régler, à l'aide d'un curseur 121 ou similaire, la pression intravésicale admissible dans l'organe 2 pendant l'intervention. A titre d'exemple, on peut prévoir une correction étagée telle que la pression effective dans la zone d'intervention corresponde à des valeurs maximales s'échelonnant entre 5 et 60 cm de colonne d'eau.

Le signal de comparaison apparaissant après traitement sur une ligne de sortie 122 de l'unité de comparaison 118 commande, éventuellement à travers un organe 123 de couplage opto-électronique, une unité 124 de commande de la pompe 108. Cette unité 124 de commande de la pompe peut avantageusement être équipée d'un poussoir 126 de commande de purge. Un indicateur optique 140, par exemple du type à galvanomètre, peut être monté sur la sortie du manomètre 112 ou du soustracteur 114 pour donner à l'opérateur une indication de la pression effective mesurée.

Selon une des caractéristiques avantageuses du dispositif, le signal de la mesure de la pression instantanée apparaissant sur la ligne 117 est corrigé pour tenir compte des sections de passage des conduits d'aspiration, notamment de la section hydraulique équivalente du canal 106 ainsi que du débit d'évacuation, ces deux paramètres créant une dépression qui est ajoutée au signal de mesure de la ligne 117.

Le signal de correction apparaît sur une ligne d'entrée additionnelle 127 de l'unité de comparaison 118. La ligne 127 provient d'un circuit de correction 129 qui prend en compte les sections de passage du canal 106. Le circuit 129 est équipé d'un organe de réglage 134, par exemple du type à curseur, ce dernier se déplaçant entre des bornes correspondant respectivement à diverses sections de passage. Le circuit de correction 129 est piloté par un signal qui apparaît à son entrée 135 et qui provient d'un inverseur 128. Cet inverseur peut occuper deux positions, à savoir une première position dans laquelle il est relié par une borne de liaison 132 au dispositif d'injection contrôlée, décrit en référence à la fig. 1, et délivrant à cette borne un signal représentatif dudit débit d'injection (au cas où un tel dispositif est mis en service) et

7

une seconde position dans laquelle il est relié à un curseur 131 d'un circuit correcteur 130 dont les bornes correspondent à différents débits d'injection que l'opérateur sélectionne selon le débit effectif, dans le cas d'une injection de liquide par simple gravité, l'affichage du débit se faisant dans ce cas par affichage de la hauteur du conteneur du liquide d'injection, le niveau du liquide de ce conteneur étant maintenu constant, par exemple par la suspension du conteneur à un ressort convenablement choisi.

Comme le montre la figure, le dispositif d'évacuation représenté peut comporter, le cas échéant, un circuit correcteur additionnel 133 prenant en compte la température des moyens sensibles à la pression, elle-même fonction notamment de la température ambiante, de celle du liquide ainsi que celle du manomètre.

Bien que le dispositif puisse être utilisé indépendamment du dispositif d'injection, notamment en combinaison avec un système d'injection par gravité, ce dispositif trouve son utilisation préférée dans les interventions sous irrigation continue dans lesquelles la pression et le débit d'irrigation sont contrôlés et constants. En fonctionnement, tant que la pression mesurée par le manomètre 112 et corrigée par les circuits 115, 129, 133 et 130 est inférieure ou égale à la pression d'opération affichée par l'opérateur sur le circuit 120, la pompe 108 est laissée à l'arrêt. A l'inverse, quand cette pression est atteinte ou dépassée, la pompe 108 est mise en route.

Dans le cas où le dispositif est utilisé avec un dispositif d'injection inerte par gravité, il constitue un régulateur grâce au contrôle permanent de la pression. Dans ce cas, l'unité de comparaison 118 peut avantageusement utiliser l'information de débit délivrée par un moyen sensible au débit 141, implanté sur le conduit 107 en amont du bocal intermédiaire 109. Les informations de débit délivrées par ledit moyen 141, connu en soi, sur une ligne 142 ainsi que l'information de pression délivrée par l'unité 114 sur la ligne 117 permettront à l'unité de comparaison 118 de corriger le débit d'aspiration pour que la pression dans la zone d'intervention garde une valeur constante sélectionnée au moyen du curseur 121 du circuit de réglage 120.

Dans le cas où ce dispositif d'évacuation contrôlée est utilisé avec un dispositif d'injection contrôlée, il fournit à ce dernier une sécurité supplémentaire. Dans ce cas, l'unité de comparaison 118 peut avantageusement utiliser l'information de pression dans la zone d'intervention délivrée par le dispositif d'injection sur la borne de raccordement 132A pour réguler le débit d'aspiration afin de maintenir ladite pression à une valeur constante sélectionnée au niveau du dispositif d'injection.

Enfin, le dispositif peut également être utilisé dans les interventions à irrigation intermittente en cas d'utilisation d'un endoscope à simple courant disposant d'un robinet d'évacuation, c'est-à-dire de deux robinets (par exemple une gaine à valves centrales). Dans ce cas, la commande d'évacuation est effectuée par l'unité de comparaison 118 qui assure le rôle d'un organe de sécurité.

Alors que l'injection au cours des interventions endoscopiques en urologie était jusqu'à ce jour obtenue par gravité, entraînant une connaissance et une maîtrise toute approximative, voire aléatoire, des paramètres hydrauliques, à savoir du volume, du débit et de la pression, l'appareil décrit ci-dessus permet par une injection à régulation électronique, éventuellement complétée par une évacuation active et contrôlée, de supprimer les contraintes et les aléas mentionnés précédemment en permettant à tout instant une connaissance parfaite et précise ainsi qu'une maîtrise complète des paramètres hydrauliques. Il en résulte une sécurité pour le malade par l'élimination totale des surpressions et de leurs complications et une amélioration considérable des conditions opératoires pour le chirurgien en lui procurant une visibilité maximale dans la zone d'intervention et en lui permettant la commande instantanée de l'irrigation. En particulier, l'appareil permet d'améliorer la visibilité dans la zone d'intervention, et par conséquent d'augmenter le confort opératoire. Le chirurgien étant déchargé des problèmes de contrôle et de réglage, il peut consacrer une partie de son temps plus importante à l'opération proprement dite et rendre son intervention plus sûre et plus efficace.

En résumé, l'injection à régulation électronique utilisée en irrigation intermittente permet dans toute intervention endoscopique :

1. La sélection des paramètres hydrauliques de l'injection :
— pression maximale du liquide dans la zone d'intervention
— débit instantané du liquide injecté
— volume maximal du liquide à injecter par cycle.
2. L'affichage permanent optique et/ou sonore de la valeur instantanée de ces paramètres :
— pression instantanée dans la zone d'intervention
— débit instantané du liquide injecté
— volume du liquide injecté depuis le début du cycle et depuis le début de l'intervention.
3. La commande instantanée par pédales de l'injection du liquide et du passage du courant électrique opératoire.
4. La correction du niveau de table c'est-à-dire la compensation des variations, décidées par l'opérateur, de la hauteur de la table d'opération, donc de l'organe opéré.

L'injecteur à régulation électronique utilisé en irrigation continue permet d'obtenir les mêmes résultats que ceux mentionnés ci-dessus. Alors qu'en mode d'irrigation intermittente, l'intervention proprement dite n'est réalisée que pendant la phase d'injection de liquide, en irrigation continue

EP 0 224 487 B1

l'opération s'effectue pendant toute la durée de l'irrigation, l'évacuation s'effectuant soit à travers un conduit spécial du résectoscope, soit à travers un trocard vésical.

L'évacuation à régulation électronique permet :

1. La sélection de la pression dans la zone d'intervention, notamment les interventions sous basse pression,

2. La régulation automatique des paramètres hydrauliques de l'aspiration :
— pression dans la zone d'intervention constante,
— débit d'aspiration égal au débit d'injection, quel que soit ce débit d'injection,
— volume vésical constant.

3. L'affichage permanent de la valeur instantanée de l'aspiration.

4. La correction du niveau de table.

La présente invention n'est pas limitée aux formes de réalisation décrites et illustrées par les figures mais peut subir différentes modifications et présenter diverses variantes évidentes pour l'homme de l'art.


**Revendications**

1. Appareil d'irrigation contrôlée des cavités et conduits naturels du corps humain, notamment pour endoscope, en particulier pour endoscope urologique, comportant au moins un canal pour injecter soit de façon intermittente, soit de façon continue un liquide d'irrigation dans une cavité et/ou un conduit corporel dits zone d'intervention, de manière à permettre aussi bien des interventions en irrigation intermittente qu'en irrigation continue, cet appareil comportant un réservoir contenant le liquide d'irrigation, un conduit de liaison pour raccorder ce réservoir audit canal, une pompe d'alimentation montée sur le conduit de liaison pour approvisionner ledit canal en liquide d'irrigation, des premiers moyens sensibles à la pression raccordés audit conduit de liaison en aval de la pompe pour mesurer la pression instantanée régnant dans ce conduit au niveau de ces moyens sensibles à la pression et pour émettre un signal de mesure représentatif de cette pression, ainsi que des moyens pour définir une pression maximale acceptable dans ladite zone d'intervention, caractérisé en ce qu'il comporte des moyens de correction pour pondérer ledit signal de mesure représentatif de la pression en fonction d'une part des pertes de charge et d'autre part de la différence de niveau qui existent entre lesdits moyens sensibles à la pression et ladite zone d'intervention pour déterminer la valeur de la pression instantanée au niveau de la zone d'intervention, et des moyens pour contrôler l'injection de liquide d'irrigation de telle manière que ladite pression dans la zone d'intervention soit à chaque instant inférieure ou égale à ladite pression maximale.

2. Appareil selon la revendication 1, caractérisé en ce que lesdits moyens pour pondérer le signal en fonction desdites pertes de charge comportent un circuit correcteur (14C) couplé à un circuit réglable de correction (22A) pourvu d'un organe de réglage (23A), ce circuit réglable de correction et cet organe de réglage étant agencés pour délivrer au circuit correcteur (14C) des signaux distincts correspondant respectivement à des sections hydrauliques équivalentes différentes du canal intérieur (6) de l'endoscope, ces sections hydrauliques équivalentes différentes étant sélectionnées au moyen dudit organe de réglage.

3. Appareil selon la revendication 2, caractérisé en ce que lesdits moyens pour pondérer le signal en fonction desdites pertes de charge comportent une unité de traitement des signaux (32) agencée pour recevoir un signal de consigne de débit d'injection d'un circuit (57) sélecteur du débit d'injection et pourvu d'un organe de réglage (58), cette unité de traitement de signaux (32) étant également agencée pour recevoir un signal de débit transmis par un élément (30) activé par le liquide propulsé par la pompe (9) et pour fournir un signal de correction au circuit correcteur (14C), ce signal de correction étant fonction du débit d'injection mesuré régulé selon ledit signal de consigne de débit.

4. Appareil selon la revendication 1, caractérisé en ce que lesdits moyens pour pondérer le signal en fonction de la différence de niveau, comportent un circuit correcteur (14B) couplé à un circuit réglable de correction (22), pourvu d'un organe de réglage (23), ce circuit réglable de correction et cet organe de réglage étant agencés pour délivrer au circuit correcteur (14B) des signaux distincts correspondant respectivement à des différences de niveau distinctes entre lesdits moyens sensibles à la pression (10) et ladite zone d'intervention, ces différences de niveau distinctes étant affichées au moyen dudit organe de réglage.

5.. Appareil selon la revendication 1, caractérisé en ce que lesdits moyens pour définir une pression maximale acceptable dans la zone d'intervention comportent un circuit de réglage (20) pourvu d'un organe de réglage (21), ce circuit et cet organe de réglage étant agencés pour délivrer à une première entrée d'un comparateur (18) des signaux distincts correspondant respectivement à différentes valeurs de ladite pression maximale acceptable dans la zone d'intervention, ces différentes valeurs étant sélectionnées au moyen dudit organe de réglage.

6. Appareil selon la revendication 1, caractérisé en ce qu'il comporte au moins un dispositif d'affichage lumineux (16C) conçu pour visualiser la pression instantanée dans la zone d'intervention.

9

7. Appareil selon la revendication 1, caractérisé en ce qu'il comporte un dispositif d'affichage lumineux (16A) pour visualiser la pression instantanée mesurée par lesdits premiers moyens (10) sensibles à la pression.

8. Appareil selon la revendication 1, caractérisé en ce qu'il comporte un dispositif d'affichage lumineux (16B) pour visualiser la pression instantanée dans le conduit (7).

9. Appareil selon la revendication 3, caractérisé en ce que l'élément (30) est agencé pour émettre un signal représentatif du débit de liquide injecté et en ce que l'unité de traitement (32) est connectée à au moins un compteur de volume du liquide délivré par la pompe (9) et au circuit correcteur (14C).

10. Appareil selon la revendication 9, caractérisé en ce qu'il comporte deux compteurs (35, 36) dont le premier (35) est agencé pour mesurer le volume de liquide délivré depuis le début d'un cycle en cours, en cas d'une irrigation intermittente, et dont le second (36) est agencé pour mesurer le volume de liquide délivré depuis le début de l'intervention quel que soit le mode d'irrigation.

11. Appareil selon la revendication 1, caractérisé en ce qu'il comporte un circuit de sélection (43) pourvu d'un organe de réglage (44) agencé pour émettre des signaux à un comparateur (41), ces signaux correspondant à différents volumes de liquide à délivrer pendant une intervention ou un cycle en cours, ces différents volumes étant sélectionnés au moyen dudit organe de réglage.

12. Appareil selon les revendications 10 et 11, caractérisé en ce que ledit comparateur (41) comporte une première entrée (42) pour lesdits signaux provenant du circuit de sélection (43) et correspondant auxdits différents volumes de liquide, et une seconde entrée (40) pour des signaux émis par ledit premier compteur (35).

13. Appareil selon la revendication 10, caractérisé en ce que chacun desdits premier et second compteur (35, 36) est respectivement associé à un premier et un second dispositif d'affichage lumineux (37, 38).

14. Appareil selon la revendication 10, caractérisé en ce qu'il comporte un premier dispositif de remise à zéro (52) et un second dispositif de remise à zéro (53), ces dispositifs étant respectivement associés au premier compteur (35) et au second compteur (36), et en ce que le premier dispositif de remise à zéro (52) est agencé pour agir sur un comparateur (41) couplé à une unité de commande (46) d'une électrovanne (48) interposée sur le conduit (7), pour arrêter l'injection du liquide lorsque le volume prédéterminé a été injecté dans la zone d'intervention.

15. Appareil selon la revendication 9, caractérisé en ce qu'il comporte deux circuits d'interface (32B et 32D) conçus pour amplifier et adapter respectivement les signaux représentatifs du débit et de la pression instantanée dans la zone d'intervention, et des bornes de raccordement (32C et 32E) pour connecter lesdits circuits d'interface à un dispositif d'évacuation actif contrôlé.

16. Appareil selon la revendication 15, dans lequel ledit dispositif d'évacuation actif contrôlé comporte un conduit (107) pour évacuer le liquide de la zone d'intervention, une pompe (108) d'évacuation montée sur ce conduit, et des seconds moyens sensibles à la pression raccordés audit conduit en amont de ladite zone d'intervention, caractérisé en ce que ledit dispositif comporte des moyens de correction pour pondérer le signal de mesure émis par lesdits seconds moyens sensibles à la pression en fonction, d'une part des pertes de charge et d'autre part de la différence de niveau qui existent entre lesdits seconds moyens sensibles à la pression et ladite zone d'intervention et des moyens pour contrôler l'évacuation du liquide d'irrigation de telle manière que ladite pression dans la zone d'intervention soit à chaque instant inférieure ou égale à ladite pression maximale.

17. Appareil selon la revendication 16, caractérisé en ce que lesdits moyens de correction pour pondérer le signal de mesure émis par lesdits seconds moyens sensibles à la pression en fonction des pertes de charge comportent une unité de comparaison et de traitement (118) et un circuit de correction (129) équipé d'un organe de réglage (134), ce circuit et cet organe étant agencés pour délivrer à l'unité (118) des signaux distincts correspondant respectivement à des sections hydrauliques équivalentes différentes du canal (106), ces sections hydrauliques équivalentes différentes étant sélectionnées au moyen dudit organe de réglage.

18. Appareil selon la revendication 16, caractérisé en ce que lesdits moyens pour pondérer le signal en fonction desdites pertes de charge comportent un circuit correcteur (130) pourvu d'un organe sélecteur (131), ce circuit correcteur et cet organe sélecteur étant agencés pour sélectionner les débits prédéterminés de liquide évacué de la zone d'intervention.

19. Appareil selon la revendication 16, caractérisé en ce que lesdits moyens pour pondérer le signal en fonction desdites pertes de charge comportent une borne de liaison (132) connectée à un circuit de correction (129) équipé d'un organe de réglage agencé pour prendre en compte un signal représentatif du débit instantané du dispositif d'injection.

20. Appareil selon la revendication 16, caractérisé en ce que lesdits moyens pour pondérer le signal en fonction de la différence de niveau comportent un circuit de réglage de correction (115) pourvu d'un organe sélecteur (116), ce circuit et cet organe étant agencés pour délivrer à un circuit correcteur (114) des signaux distincts correspondant respectivement à des différences de niveau distinctes entre lesdits seconds moyens sensibles à la pression (112) et la zone d'intervention, ces différences de niveau distinctes étant affichées au moyen dudit organe sélecteur.

**Claims**

1. Apparatus for controlled irrigation of the natural cavities and tubes of the human body by endoscopy, particularly an urological endoscope wherein at least one duct is provided to inject an irrigation fluid intermittently or continuously in a corporeal cavity and/or a corporeal intervention area whereby to permit interventions both with intermittent and continuous irrigations, said apparatus comprising : a tank containing irrigation fluid ; a joining conduit connecting said tank to said at least one duct ; a supply pump mounted on said joining conduit for supplying said duct with irrigation fluid ; first means sensitive to pressure, connected below said pump to said joining conduit, for measuring the instant pressure in said conduit at the level of said first means and for emitting a measuring signal representing this pressure ; second means for defining an acceptable maximum pressure in said intervention area ; correcting means for equalizing said measuring signal according to pressure losses and to the difference in level existing between said first means and said intervention area, whereby to determine the value of said instant pressure at the level of said intervention area ; and means for controlling the injection of irrigation fluid in such manner that said pressure in said intervention area is at all times not greater than said maximum pressure.

2. Apparatus according to claim 1, wherein said correcting means for equalizing said measuring signal in accordance with said pressure losses comprises a correcting circuit (140) coupled with a controllable correcting circuit (22A) having a control organ (23A), said controllable correcting circuit and said control organ being adapted to deliver to said correcting circuit (140) distinct signals which correspond respectively to hydraulically equivalent cross sections of the inner duct of said endoscope, said different hydraulically equivalent cross sections being selected by means of said control organ.

3. Apparatus according to claim 2, wherein said correcting means for equalizing said measuring signal according to said pressure losses includes a signal processing unit (32), adapted to receive an order signal representing said different hydraulically cross sections of said inner duct of said endoscope, said order signal being transmitted by a selecting unit (57) provided with a regulating organ (58), said signal processing unit (32), being also adapted to receive a flow rate signal transmitted by an element (30), which is actuated by fluid propelled by said pump (9), and to emit a correcting signal to said correcting circuit (14B), said correcting signal being in accordance with the injection flow rate which is measured and regulated according to said control signal of the flow rate.

4. Apparatus according to claim 1, wherein said correcting means for equalizing said measuring signal according to said difference in level includes a correcting circuit (14B), coupled with a controllable correcting circuit (22), having a control organ (23), said controllable correcting circuit and said control organ being adapted to emit to said correcting circuit (14B), distinct signals which correspond respectively to distinct level differences between said first means (10), and said intervention area, said distinct level differences being displayed by means of said control organ.

5. Apparatus according to claim 1, wherein said second means includes a control circuit (20), having a control organ (21), said control circuit and said control organ being adapted to emit to a first input of a comparator (18), distinct signals which correspond respectively to different values of said acceptable maximum pressure in said intervention area, said different values being selected by means of said control organ.

6. Apparatus according to claim 1, including at least one optical display device (160) adapted for visualizing the instant pressure in said intervention area.

7. Apparatus according to claim 1, including an optical display device (16A), for visualizing the instant pressure which is measured by said first means (10).

8. Apparatus according to claim 1, including an optical display device (16B), for visualizing the instant pressure in said joining conduit (7).

9. Apparatus according to claim 3, wherein said element (30) actuated by the fluid propelled by said pump is adapted for emitting a signal which represents the flow rate of the injected fluid, and wherein said signal processing unit (32) is connected to at least one counter of the fluid volume delivered by said pump (9), to said correcting circuit (14C).

10. Apparatus according to claim 9, including two counters (35, 36), the first of which (35), is adapted for measuring the volume of fluid delivered since the beginning of a current cycle in the case of intermittent irrigation, and the second of which (36), is adapted for measuring the volume of fluid delivered since the beginning of intervention by either mode of irrigation.

11. Apparatus according to claim 1, including a preselection unit (43), provided with a control organ (44) adapted for emitting signals to a comparator (41), said signals corresponding to different fluid volumes which are to be delivered during an intervention or a current cycle, said different volumes being selected by means of said control organ.

12. Apparatus according to claims 10 and 11, wherein said comparator (41) includes a first input (42) for said signals which proceeds from said preselection unit (43), and which corresponds to said different volumes of fluid, and a second input (40), for signals which are emitted by said first counter (35).

13. Apparatus according to claim 10, wherein each of said first and second counters (35, 36), is associated respectively with a first and a second visual display device (37, 38).

14. Apparatus according to claim 10, including a first zero resetting device (52), and a second zero

11

resetting device (53), said resetting devices being associated respectively with said first counter (35), and with said second counter (36), and wherein said first zero resetting device (52) is adapted to act on a comparator (41), coupled with a control unit (46), of an electric valve (48), interposed in said joining conduit (7), so as to stop the injection of fluid when a predetermined volume has been injected into said intervention area.

15. Apparatus according to claim 9, including two adaptation circuits (32B, 32D), designed to amplify and adapt respectively the signals representative of the flow rate and of the instant pressure in said intervention area, and terminal connections (32C, 32E), for communicating said adaptation circuits with an active and controlled withdrawing device.

16. Apparatus according to claim 15, wherein said active and controlled withdrawing device includes a conduit (107), for withdrawing the fluid of said intervention area, a withdrawing pump (108), mounted on said conduit, second means sensitive to pressure connected to said conduit above said intervention area, said withdrawing device including correcting means for equalizing the measuring signal emitted by said first means according to said pressure losses on one hand and to said difference in level on the other hand, which exist between said first means and said intervention area, and means for controlling the withdrawing of the irrigation fluid, whereby said pressure in said intervention area is at any instant not greater than said maximum pressure.

17. Apparatus according to claim 16, wherein said correcting means for equalizing the measuring signal emitted by said second means includes a comparison and processing unit (118), and a correcting circuit (129), equipped with a control organ (134), said correcting circuit and said organ being adapted to emit so said comparison and processing unit (118), different signals corresponding respectively to different hydraulically equivalent cross sections of said duct (106) in said endoscope, said different hydraulically equivalent cross sections being preselected by means of said control organ.

18. Apparatus according to claim 16, wherein said correcting means for equalizing said measuring signal according to pressure losses includes a correcting circuit (130), having a preselecting organ (131), said correcting circuit and said preselecting organ being adapted to preselect the flow rates of fluid withdrawn from said intervention area.

19. Apparatus according to claim 16, wherein said correcting means for equalizing said measuring system according to said pressure losses includes a terminal connection (132), connected with a correcting circuit (129) wich is equipped with a control organ adapted to take into account a signal representative of the instant flow rate of said injection device.

20. Apparatus according to claim 16, wherein said correcting means for equalizing the signal according to said difference in level includes a controllable correcting circuit (115) equipped with a preselecting organ (116), said circuit and said organ being adapted to emit to a correcting circuit (114), distinct signals which respectively correspond to distinct level differences between said second means (112), and said intervention area, said distinct level differences being displayed by means of said preselecting organ.


## Patentansprüche

1. Steuergerät für eine Anordnung zum Spülen menschlicher Körperhöhlen, insbesondere im Zusammenhang mit einem Endoskop, speziell für ein urologisches Endoskop, welches mindestens eine Zuführungsleitung aufweist, um entweder periodisch oder kontinuierlich eine Spülflüssigkeit in eine Höhle und/oder eine Röhre des Körpers, hier Interventionszone genannt, einzuführen, derart, daß sowohl Eingriffe bei periodischer Spülung als auch bei kontinuierlicher Spülung möglich sind, wobei dieses Gerät folgendes aufweist : Ein die Spülflüssigkeit enthaltendes Reservoir, eine Verbindungsleitung, um dieses Reservoir an diese Zuführung anzuschließen, eine in der Verbindungsleitung angeordnete Beschickungspumpe, um die Zuführungsleitung mit Spülflüssigkeit zu versorgen, erste an die Verbindungsleitung nach der Pumpe angeschlossene Druckmesseinrichtungen, um den in dieser Leitung in Höhe dieser Druckmesseinrichtungen herrschenden momentanen Druck zu messen und um ein für diesen Druck repräsentatives Meßsignal abzugeben, sowie Einrichtungen, um einen maximal zulässigen Druck in der Interventionszone festzulegen, dadurch gekennzeichnet, daß es Korrektureinrichtungen aufweist, um das für den Druck repräsentative Meßsignal in Abhängigkeit einerseits vom Druckverlust und andererseits vom Niveauunterschied zwischen den Druckmesseinrichtungen und der Interventionszone zu berichtigen, um die Größe des augenblicklichen Druckes in der Interventionszone zu bestimmen, und schließlich Einrichtungen, um die Zuführung von Spülflüssigkeit derart zu kontrollieren, daß der Druck in der Interventionszone jederzeit niedriger oder gleich dem Maximaldruck ist.

2. Gerät nach Anspruch 1, dadurch gekennzeichnet, daß die Einrichtungen zum Berichtigen des Signals in Abhängigkeit der Druckverluste eine Kompensatorschaltung (14C) aufweisen, die mit einer regelbaren, mit einem Regulierorgan (23A) versehenen, Korrekturschaltung (22A) gekoppelt ist, wobei diese regulierbare Korrekturschaltung und dieses Regulierorgan dazu dient, in die Kompensatorschaltung (14C) verschiedene, unterschiedlichen hydraulischen äquivalenten Abschnitten der inneren Zuführung (6) des Endoskopes entsprechende Signale zu geben, dabei sind diese unterschiedlichen hydraulischen äquivalenten Abschnitte mit Hilfe des Regulierorgans auswählbar.

3. Gerät nach Anspruch 2, dadurch gekennzeichnet, daß die Einrichtungen zum Ausgleichen des Signales in Abhängigkeit der Druckverluste eine Signalverarbeitungseinheit (32) aufweist, die so ausgelegt ist, daß sie ein Einstellwertsignal für die Zuführmenge von einer Wählschaltung (57) für die Zuführmenge erhält und mit einem Regulierorgan (58) versehen ist, wobei diese Signalverarbeitungseinheit (32) auch ausgelegt ist, um ein Signal für die Durchsatzmenge zu empfangen, welches von einem Element (30) kommt, das über die von der Pumpe (9) geförderten Flüssigkeiten aktiviert ist und um an die Kompensatorschaltung (14C) ein Korrektursignal abzugeben, wobei dieses Korrektursignal von der gemessenen und nach dem Einstellwert für die Durchsatzmenge regulierten Zuführmenge abhängt.

4. Gerät nach Anspruch 1, dadurch gekennzeichnet, daß die Einrichtungen zum Ausgleichen des Signales in Abhängigkeit des Niveauunterschiedes eine Kompensatorschaltung (14B) aufweisen, die an eine einstellbare, mit einem Regulierorgan (23) versehene Korrekturschaltung (22) gekoppelt ist, wobei diese regulierbare Korrekturschaltung und dieses Regulierorgan so eingerichtet sind, daß sie an die Kompensatorschaltung (14B) verschiedene Signale abgeben, die den verschiedenen Niveauunterschieden zwischen den Druckmesseinrichtungen (10) und der Interventionszone entsprechen und diese verschiedenen Niveauunterschiede mittels des Regulierorgans anzeigbar sind.

5. Gerät nach Anspruch 1, dadurch gekennzeichnet, daß die Einrichtungen zur Festlegung eines in der Interventionszone zulässigen Maximaldruckes eine Regulierschaltung (20) aufweisen, die mit einem Regulierorgang (21) ausgerüstet ist, wobei diese Schaltung und dieses Regulierorgan so eingerichtet sind, daß sie an einen ersten Eingang eines Differenziergliedes (18) verschiedene, jeweils unterschiedlichen Werten des in der Interventionszone zulässigen maximalen Druckes zugeordnete Signale abgeben, wobei diese unterschiedlichen Werte mittels des Regulierorgans auswählbar sind.

6. Gerät nach Anspruch 1, dadurch gekennzeichnet, daß es eine Leuchtanzeige (16A) aufweist, um den von den ersten Druckmeßeinrichtungen (10) gemessenen momentanen Drücke sichtbar zu machen.

7. Gerät nach Anspruch 1, dadurch gekennzeichnet, daß es eine Leuchtanzeige (16A) aufweist, um den von den ersten Druckmeßeinrichtungen (10) gemessenen momentanen Druck sichtbar zu machen.

8. Gerät nach Anspruch 1, dadurch gekennzeichnet, daß es eine Leuchtanzeige (16B) aufweist, um den momentanen Druck in der Zuleitung (7) sichtbar zu machen.

9. Gerät nach Anspruch 3, dadurch gekennzeichnet, daß das Element (30) dazu dient, ein der zugeführten Flüssigkeitsmenge entsprechendes Signal abzugeben und daß die Signalverarbeitungseinheit (32) mit mindestens einem Zähler für die von der Pumpe (9) geförderte Flüssigkeitsmenge und mit der Kompensatorschaltung (14C) verbunden ist.

10. Gerät nach Anspruch 9, dadurch gekennzeichnet, daß es zwei Zähler (35, 36) aufweist, von denen der erste (35) dazu dient, die im Falle einer periodischen Spülung vom Beginn eines laufenden Zyklus an geförderte Flüssigkeitsmenge zu messen, und der zweite (36) dazu dient, die vom Beginn des Eingriffes an geförderte Flüssigkeitsmenge zu messen, gleichgültig um welches Spülverfahren es sich handelt.

11. Gerät nach Anspruch 1, dadurch gekennzeichnet, daß es eine mit einem Regulierorgan (44) versehene Wählschaltung (43) aufweist, die dazu dient, an ein Differenzierglied (41) Signale abzugeben, wobei diese Signale unterschiedlichen Flüssigkeitsmengen entsprechen, die während eines Eingriffes oder eines laufenden Zyklus zu fördern sind, und diese unterschiedlichen Flüssigkeitsmengen mittels des Regulierorgans wählbar sind.

12. Gerät nach den Ansprüchen 10 und 11, dadurch gekennzeichnet, daß das Differenzierglied (41) einen ersten Eingang (42) aufweist für die von der Wählschaltung (43) kommenden und den unterschiedlichen Flüssigkeitsmengen entsprechenden Signale, und einen zweiten Eingang (40) für die vom ersten Zähler (35) abgegebenen Signale.

13. Gerät nach Anspruch 10, dadurch gekennzeichnet, daß sowohl der erste als auch der zweite Zähler (35, 36) jeweils mit einer ersten und einer zweiten Leuchtanzeige (37, 38) verbunden ist.

14. Gerät nach Anspruch 10, dadurch gekennzeichnet, daß es eine erste Null-Stellungseinrichtung (52) und eine zweite Null-Stellungseinrichtung (53) aufweist, wobei diese Einrichtungen jeweils mit dem ersten Zähler (35) und dem zweiten Zähler (36) verbunden sind, und daß die erste Null-Stellungseinrichtung (52) dazu dient, auf ein Differenzierglied (41) zu wirken, das an eine Steuereinheit (46) eines Elektroventils (48) in der Zuleitung (7) gekoppelt ist, um die Zuführung von Flüssigkeit zu unterbrechen, wenn die vorbestimmte Menge der Interventionszone zugeführt ist.

15. Gerät nach Anspruch 9, dadurch gekennzeichnet, daß es zwei Schnittstellenleitungen (32B, 32D) aufweist, die dazu dienen, die der Fördermenge und dem Momentandruck in der Interventionszone entsprechenden Signale zu verstärken und anzupassen, sowie Anschlußklemmen (32C, 32E) zur Verbindung der Schnittstellenleitungen mit einer Einrichtung für kontrollierten aktiven Ablauf.

16. Gerät nach Anspruch 15, bei welchem die Einrichtung für kontrollierten aktiven Ablauf eine Leitung (107) für das Ablaufen der Flüssigkeit aus der Interventionszone aufweist, sowie eine in diese Leitung eingebaute Ableerpumpe (108), und zweite an diese Leitung vor der Interventionszone angeschlossene Druckmesseinrichtungen, dadurch gekennzeichnet, daß die Einrichtung Korrekturmittel aufweist, um das von den zweiten Druckmesseinrichtungen abgegebene Meßsignal auszugleichen, einerseits in Abhängigkeit der Druckverluste und andererseits in Abhängigkeit des Höhenunterschiedes zwischen den zweiten Druckmesseinrichtungen und der Interventionszone, sowie Mittel zum Überwachen des Ablaufes der Spülflüssigkeit derart, daß der Druck in der Interventionszone in jedem Augenblick niedriger oder gleich dem Maximaldruck ist.

17. Gerät nach Anspruch 16, dadurch gekennzeichnet, daß die Korrekturmittel zum Ausgleichen des von den zweiten Druckmesseinrichtungen abgegebenen Meßsignales in Abhängigkeit der Druckverluste eine Vergleichs- und Verarbeitungseinheit (118) aufweisen sowie eine mit einem Regulierorgan (134) ausgestattete Korrekturschaltung (129), wobei diese Schaltung und dieses Organ dazu dienen, an die Einheiten (118) verschiedene Signale abzugeben, die jeweils zu unterschiedlichen entsprechenden Abschnitten der Leitungen (106) gehören, wobei diese unterschiedlichen entsprechenden Abschnitte mittels des Regulierorgans wählbar sind.

18. Gerät nach Anspruch 16, dadurch gekennzeichnet, daß die Mittel zum Ausgleichen des Signales in Abhängigkeit der Druckverluste eine mit einem Wählorgan (131) ausgestattete Korrekturschaltung (130) aufweisen, wobei diese Korrekturschaltung und dieses Wählorgan dazu dienen, die vorbestimmten aus der Interventionszone abzulassenden Flüssigkeitsmengen zu wählen.

19. Gerät nach Anspruch 16, dadurch gekennzeichnet, daß die Mittel zum Ausgleichen des Signales in Abhängigkeit der Druckverluste eine Anschlußklemme (132) aufweisen, welche mit einer mit einem Regulierorgan ausgestatteten Korrekturschaltung (129) verbunden ist, die dazu dient, ein die momentane Fördermenge der Zuführeinrichtung angebendes Signal zu berücksichtigen.

20. Gerät nach Anspruch 16, dadurch gekennzeichnet, daß die Mittel zum Ausgleichen des Signales in Abhängigkeit des Höhenunterschiedes eine mit einem Wählorgan (116) ausgestattete Regel- und Korrekturschaltung (115) aufweisen, wobei diese Schaltung und dieses Organ dazu dienen, an eine Kompensatorschaltung (114) verschiedene Signale entsprechend jeweils den Unterschieden von verschiedenen Niveaus zwischen den zweiten Druckmesseinrichtungen (112) und der Interventionszone abzugeben, wobei diese Unterschiede der verschiedenen Niveaus mittels des Wählorgans anzeigbar sind.

FIG.1

EP 0 224 487 B1

FIG.2